# EUROPEAN PATENT SPECIFICATION

(11) **EP 4 368 583 B1**
(45) Date of publication and mention of the grant of the patent: **27.08.2025**
(21) Application number: 23209040.7
(22) Date of filing: 10.11.2023
(51) Int. Cl.: C02F 1/54, C02F 1/66, B01D 47/14, B01D 53/14, C02F 1/28, C02F 101/10, C02F 101/32, C02F 103/18

(54) **COMPOSITION AND METHOD FOR REDUCING ODORS IN WET AIR SCRUBBERS**
ZUSAMMENSETZUNG UND VERFAHREN ZUR GERUCHSVERMINDERUNG IN NASSLUFTWÄSCHERN
COMPOSITION ET PROCÉDÉ POUR RÉDUIRE LES ODEURS DANS DES ÉPURATEURS D'AIR HUMIDE

(30) Priority: 14.11.2022 US 202263425126 P; 15.03.2023 US 202318121836
(43) Date of publication of application: 15.05.2024
(73) Proprietor: NCH Corporation, Irving, Texas 75062 (US)
(72) Inventor: Davis, Charles L., Dallas, TX, 75208 (US); Denvir, Adrian J., Richardson, TX, 75080 (US); Delegard, Angela L., Flower Mound, TX, 75028 (US); Drewniak, Marta, Carrollton, TX, 75007 (US)
(74) Representative: Grünecker Patent- und Rechtsanwälte PartG mbB

(56) References cited:
- WO-A1-2012/106443
- US-A1- 2012 219 480
- US-A1- 2019 014 786
- SUN ZHIGUO ET AL: "Treatment of Waste Gases by Humic Acid", ENERGY & FUELS, vol. 29, no. 3, 17 February 2015 (2015-02-17), WASHINGTON, DC, US., pages 1269 - 1278, XP093111643, ISSN: 0887-0624, DOI: 10.1021/ef502299k

## Description

### CROSS-REFERENCE TO RELATED APPLICATION

This application claims the benefit of U.S. provisional patent application No. 63/425,126 filed November 14, 2022.

### Background of the Invention

### 1. Field of the Invention

This invention relates to a composition and method for reducing odors in wet air scrubbers, particularly in rendering facilities.

### 2. Description of Related Art

A rendering facility is a place where waste animal tissue along with industrial and household fats, oils, and grease are converted into stable and usable materials. However, in the rendering process, volatile organic compounds are released into the air as a result of the operation. These volatile organic compounds include organic and inorganic sulfur compounds, organic acids, aldehydes, ketones, along with aromatic and aliphatic organic compounds. If not operated correctly, the rendering facility can be considered an odor nuisance, especially if it is near a residential area. In the United States alone about 21 million metric tons of highly perishable and noxious organic materials are rendered each year. One of the methods that is employed to minimize the amount of waste expelled into the air from rendering facilities is air scrubber technology, including wet air scrubbers.

Air scrubbers may, for example, comprise a tower with water flowing from the top of the tower to the bottom of the tower, with water then recycled to the top of the tower again. As air from a rendering plant is flowed through the air scrubber, VOCs may be removed from the air. Air scrubbers may use sprayed water to create an air/water interface for purification and/or use fill or packing material or media (such as plastic or stainless steel pieces) to increase air/water surface area and to decrease water flow as the air flows upwards through the scrubber. The fill or packing material is prone to fouling through accumulation of fats, oils, or greases and/or biofilm growth on the media in the scrubber, which can contribute to odors coming from the scrubber and can reduce the effectiveness of the scrubber.

A gas stream from the rendering plant is fed into an air scrubber to remove at least some of the VOCs/odor causing components before the scrubber outlet gas is released to the atmosphere. Air scrubbers use air flow and water droplets (typically with added media, such as plastic or stainless steel pieces, within the scrubber to increase air/water contact surface area) to capture the pollutant materials from the scrubber inlet gas stream and transfer them to the water exiting the bottom of the scrubber. The water may be recirculated a number of times through the scrubber until it needs to be flushed to a drain or other waste disposal system.

Various treatment compositions for treating the water in a wet scrubber are known in the art. These treatments use different chemical compounds or combination of compounds to improve efficiency, reduce fouling or biofilm growth on the media in the scrubber, and reduce the release of noxious chemicals into the atmosphere. Frequently, the treatment compositions include acids or oxidizers that have environmental drawbacks that may limit the ability to dispose of the scrubber water, may also be corrosive to components of the scrubber and drain system, and present issues with storage, handling, and shipment of the treatment compositions.

For example, U.S. Patent No. 8,753,567 patent owned by Hydro-Solutions, Inc. teaches the use of a dicarboxylic acid solution dosed at a concentration of 100-2000 ppm, with a pH range of 5-8 in the scrubber water, to remove volatile organic compounds. The dicarboxylic acids include oxalic acid, malonic acid, succinct acid, glutaric acid, and malic acid, with malic acid being preferred. The treatment composition may also include aldehydes or ketones in combination with a scale inhibitor and an oil dispersant. The treatment requires control of the pH in the wet scrubber water through the addition of mineral acids which is problematic as it involves specialty sites for storing strong acids on site and there is a hazard associated with handling these strong acids at the facility. Additionally, working with strong acids can also cause corrosion problems within the water handling system if not monitored correctly. The commercial product corresponding the '567 patent is known as React Ox.

Diversey, Inc. also has several published applications related to wet scrubber treatment compositions. For example, U.S. Patent Application Publication No.2012/0219480 A1 discloses Components designated by letters A-D that are useable for wet scrubber treatment. Component A (commercially available as ReNew A) is a surfactant (preferably an amphoteric/zwitterionic surfactant) and an enzyme. The enzymes in Component A are known to be easily inactivated with changes in temperature, pH, or other storage conditions. Component B (commercially available as ReNew B) which can be a detersive surfactant and defoamer or a low foam surfactant (Plurafac, Pluronic, Triton, or Neodol are commercial products mentioned by name) selected from a capped alkoxylate, an ethylene oxide/proplyene oxide block co-polymer, or an alkoxylated amine or amine surfactant. Component C (commercially available as ReNew Balance) is a pH control agent including an acid (preferably citric acid), or a base, or both. Component D is a defoamer. The '480 application specifies that Components A and B have to be separately fed into the wet scrubber, they cannot be pre-mixed or combined into a single treatment composition as Component B needs to be added on a system to system basis and this ratio can vary making it not possible to have a single premixed product. In commercial practice a combination of Components A and B or Components A and C are standard treatments in the industry. However, the need to add the components separately, rather than having a pre-mixed composition, makes handling more difficult and makes it more likely that the incorrect amounts of one or the other component will be added.

As other Diversey examples, PCT Publication WO 2013/012934 A1 discloses a method of controlling biofilm in wetter scrubbers by operating a phosphate control program that uses iron oxides, calcium and magnesium ions, or aluminum ions to precipitate or co-precipitate phosphate from the water and PCT Publication WO 2012/061068 discloses the use of a tracer (triazoles) with the ReNew products so that amounts in the water can be monitored using colorimetric analysis.

U.S. Patent No. 11,198,828 discloses a method for removing hydrogen sulfide in a scrubber system with or without the use of an aqueous chelating agent. The treatment uses an aqueous solution of ferric ions (pH 6) that reacts with dissolved hydrogen sulfide gas to produce ferrous ions that are converted back to ferric ions with air and a manganese catalyst. If the pH is low enough there is no need for a chelating agent. However, the '828 patent also discloses use of chelating agents such as EDTA, DTAP, and NTA or a combination of these as part of the formula. The use of such chelating agents is not desirable from an environmental standpoint.

As another example, U.S. Patent No. 4,007,262 discloses an odor control composition where the chemical treatment is for a holding tank (not a wet scrubber) and consists of a neutral or basic transition metal salt or a lower aliphatic organic mono carboxylic acid dissolved in aqueous ammonia or an aqueous solution of strongly soluble organic amine. The treatment may also contain an oxidizing agent such as permanganate, an alkaline stable dye and fragrance. The use of these components creates environmental issues and makes storing, shipping, and handling hazardous.

U.S. Patent No. 4,902,489 discloses a method for removing sulfurous odors and sulfur compounds by washing the gas with an organic acid solution (citric, acetic, mesotartaric, and mesaconic) then treating with lignin sulfonate solution 0.05 to 1.5% by weight. This treatment targets only sulfur containing compounds and does not address the other chemicals that could contribute to the malodor and it does not address removal of the deposits of fats or other substances on the packing material in the wet air scrubber. Further, relevant documents are WO 2012/106443 A1,
US 2019/014786 A1 and SUN ZHIGUO ET AL: "Treatment of Waste Gases by Humic Acid",ENERGY & FUELS, vol. 29, no. 3, 17 February 2015 (2015-02-17), pages 1269-1278.

There is a need for a treatment composition and method that can aid in reducing odors in a wet scrubber without the use of oxidizers, strong acids, and/or environmentally undesirable chelating agents (such as EDTA, DTAP, and NTA). There is also a need for a treatment composition and method that allows for use of a premixed composition where the ingredients are compatible with each other and stable for storage. There is also a need for a treatment composition that can clean the wet scrubber fill or packing material of any fats, oils, and greases that may build up during the day to day operation of the wet air scrubber. Finally, there is a need for a treatment composition and method that are not as dependent on pH conditions in the wet scrubber water as some of the prior art treatments.

### SUMMARY OF THE INVENTION

According to the invention, treatment composition for treating water in a wet scrubber comprises a combination of surfactants in conjunction with a humic acid-based reagent to (1) help control pH, (2) remove sulfur containing compounds, and (3) eliminate the need for additional foaming control. The treatment composition reduces odors in air scrubber and cleans packing media in the air scrubber (if present).

According to the invention, the treatment composition comprises (1) at least one nonionic surfactant, (2) at least one amphoteric (zwitterionic) surfactant, and (3) a source of humic acid, preferably organic peat humus. According to another preferred embodiment, the at least one nonionic surfactant comprises at least one low foam nonionic surfactant. According to another preferred embodiment, the at least one nonionic surfactant comprises at least one low foam nonionic surfactant and at least one nonionic surfactant that is not a low foam surfactant. According to still other preferred embodiments, a treatment composition further optionally comprises a fragrance. In these preferred embodiments, the balance of the composition is preferably an aqueous solvent, most preferably DI water. Treatment compositions according to preferred embodiments are pre-mixed (at the point of manufacture/packaging) into a single, liquid composition so that individual/separate ingredients or components do not need to be added to the wet scrubber system separately or mixed at the treatment site.

According to one preferred embodiment, a treatment composition comprises (1) 1-40% total of one or more nonionic surfactants; (2) 20-40% total of one or more amphoteric (zwitterionic surfactants); and (3) 1-15% organic peat humus. According to another preferred embodiment, a treatment composition comprises 2-10% total of one or more nonionic surfactants that are not low foam. According to another preferred embodiment, a treatment composition comprises 1-30% total of one or more low foam nonionic surfactants and 2-10% total of one or more additional nonionic surfactants that are not low foam. According to still another preferred embodiment, a treatment composition further optionally comprises 1-3% of a fragrance. According to still another preferred embodiment, these ingredients are all premixed into a liquid treatment composition using 20-60% of an aqueous solvent, with the percentages being by weight of the composition.

According to still another preferred embodiment, a tracer may be added to the treatment composition to allow the amount of treatment composition in the wet scrubber water to be monitored. Tracer molcules or compouds are well known in the art.

According to one preferred method of treating a wet scrubber, a treamtent composition (preferably one according to a preferred embodiment of the invention) is added to the water in a wet scrubber to achieve a concentration of the treatment composition of around 50-1000 ppm. Additional treatment composition is periodically added to maintain this concentration level. Most preferably, the treatment composition is added to a make-up water source feeding into the wet scrubber using a commercially available chemical pump (which is likely already present in the set-up of most existing wet scrubbers to add prior art treatment compositions).

According to another preferred embodiment, the method further comprises measuring a tracer in the composition to determine the amount of composition in the wet scrubber water to ensure that the concentration is maintained within preferred levels. According to another preferred embodiment, the method further comprises measuring COD in the wet scrubber water to track the amount of treatment composition, in which case a tracer is not necessary to ensure concentrations are maintained within preferred levels.

Preferred embodiments of the compositions and method of the invention aid in reducing odors in a wet scrubber without the use of oxidizers, strong acids (mineral acids), and/or environmentally undesirable chelating agents (such as EDTA, DTAP, and NTA). Preferred embodiments improve air quality at rendering facilities by making it easier to apply as a single, pre-mixed liquid composition. Preferred embodiments are particularly effective due to the synergistic nature of the product in achieving foam breaking, pH buffering, and removal of sulfur containing products (particularly H2S). Preferred embodiments also allow for use of a pre-mixed composition where the ingredients are compatible with each other and stable for storage, eliminating the need to add separate components, or mix components at the treatment site, which can be hazardous to workers and is more likely to result in incorrect dosage. Preferred embodiments also aid in cleaning the wet scrubber fill or packing material of any fats, oils, and greases that may build up, which helps eliminate some odor sources and aids in maintaining proper functioning of the scrubber. Preferred embodiments also aid in controlling pH within the scrubber so that separate pH control treatments may not be needed, and the functionality of the treatment composition is not as dependent on pH conditions in the wet scrubber water as some of the prior art treatments, and therefore do not require tight pH control.

### BRIEF DESCRIPTION OF THE DRAWINGS

The composition and method of the invention is further described and explained in relation to the following figures wherein:
FIGS. 1A and 1B are graphs showing contaminant (acetic acid) concentration as a function of time in an air scrubber test with different prior art treatment compositions and compositions according to preferred embodiments of the invention; and
FIG. 2 is a graph showing foam height (left bars) and foam half-life (right bars) for the air scrubber test of FIG. 1.

### DESCRIPTION OF PREFERRED EMBODIMENTS

According to one preferred embodiment, a liquid treatment composition for use with a wet scrubber comprises one or more surfactants in combination with a humic-acid based reagent. Most preferably, a treatment composition comprises (1) at least one nonionic surfactant, (2) at least one amphoteric (zwitterionic) surfactant, and (3) a source of humic acid, preferably organic peat humus. According to another preferred embodiment, a treatment composition further optionally comprises a fragrance. The balance in these preferred embodiments is an aqueous solvent. The ingredients in treatment compositions according to the invention are most preferably combined into a single composition, with all ingredients being compatible with each other and storage-stable, to avoid having to add separate components/treatment compositions to the wet scrubber. Treatment compositions according to preferred embodiments provide liquid odor control for wet scrubbers to minimize the release of odors from the water and packing material in wet air scrubbers.

Most preferably, the one or more nonionic surfactants comprise (1) at least one nonionic surfactant that is not a low foam surfactant (referred to herein as a "regular nonionic surfactant" and (2) optionally at least one low foam nonionic surfactant. A low foam or low foaming surfactant is one where the foam half-life is less than the regular nonionic surfactant and it collapses faster. The regular nonionic surfactants aid in reducing surface tension and provide detergent cleaning. The optional low foam nonionic surfactants aid in surface wetting, detergency, surface tension reduction, and to provide synergistic antifoaming and foam breaking properties. The low foaming surfactant reacts synergistically with the other surfactants for soil removal as well as antifoaming tendencies.

Preferably, a treatment composition comprises around 2-40%, more preferably around 4-30% total of one or more nonionic surfactants (regular and/or low foam). According to another preferred embodiment, a treatment composition comprises around 2-10%, more preferably around 2-5% total of one or more regular nonionic surfactants. According to another preferred embodiment, a treatment composition comprises around 1-30%, more preferably around 2-25% total of one or more low foam nonionic surfactants. According to another preferred embodiment, a treatment composition comprises (1) around 2-10%, more preferably around 2-5%, and most preferably around 4% total of one or more regular nonionic surfactants and (2) around 1-30%, more preferably around 2-25%, and most preferably around 25% total of one or more low foam nonionic surfactants.

Preferred regular nonionic surfactants include Tomadol^{®} 91-6 (a linear C9-11 ethoxylated alcohol), Bio-Soft^{®} N91-6 (a linear C9-11 ethoxylated alcohol), Genapol^{®} ud-079 (undecanol, branched and linear, ethoxylated). Other regular nonionic surfactants that may be used include amides, , block polymers, alkoxylated primary and secondary alcohols, alkoxylated fatty esters, sorbitan derivatives, propoxylated and alkoxylated fatty acids, alcohols, and alkyl phenols, glycol esters, and polymeric polysaccharides. Most preferably, the regular nonionic surfactants do not include alkanolamides, amine oxides, glycerol esters, alkoxylated alkylphenols, or glycol esters. Examples of commercially available regular nonionic surfactants that may be used include: Tergitol^{™} surfactants, particularly Tergitol^{™} 15-S-7 or 15-S-9 (secondary alcohol ethoxylates) marketed by Union Carbide Corporation; Neodol^{™} surfactants, particularly Neodol^{™} 45-9, Neodol^{™} 45-7 and Neodol^{™} 45-4 (blends of C14-C15 lightly branched primary alcohols) and/or Neodol^{™} 23-6.5 (a blend of C12-C13 lightly branched primary alcohols) marketed by Shell Chemical Company; Kyro EOB (a C13-C15 ethyoxylated alcohol) marketed by The Procter & Gamble Company; and Berol^{®} 260 and Berol^{®} 266 (C9-11 linear ethoxylated alchols) marketed by Akzo Nobel. A mixture of nonionic surfactants may also be used. Most preferably, only a single nonionic surfactant (plus any low foam nonionic surfactant) is used.

Preferred low foam nonionic surfactants include nonionic ethylene oxide (EO) containing surfactants, capped alkoxylates such as capped ethoxylates, capped alcohol alkoxylates such as capped alcohol ethoxylates, EO/PO (ethylene oxide group/propylene oxide group block copolymer, fatty alcohol alkoxylates, alkoxylated amines such as ethoxylated amines, amine surfactants, low cloud point nonionic surfactants (as detailed below). Examples of commercially available low foam nonionic surfactants that may be used include Triton^{™} surfactants such as Triton^{™} DF-12, Triton^{™} CF-32 and Triton^{™} CF-10 (Dow, Midland, Mich.); Pluronic^{®} surfactants such as Pluronic^{®} 25-R-2, Pluronic^{®} N-3, Pluronic^{®} SLF-18, Pluronic^{®} L-61, and Pluronic^{®} L-62 (BASF, Florham Park, N.J.); and Plurafac^{®} surfactants such as Plurafac^{®} LF-403, Plurafac^{®} LF-101, and Plurafac^{®} LF-400 (BASF, Florham Park, N.J.). A mixture of low foam nonionic surfactants may also be used. Most preferably, only a single low foam nonionic surfactant (plus any other, non-low foam nonionic surfactant) is used. Most preferably, low foam nonionic surfactants used have a cloud point of 35-50 °C.

One or more amphoteric (zwitterionic) surfactants aid in providing additional surface tension reduction, detergent cleaning, and wetting ability. Preferably, a treatment composition comprises around 20-40%, more preferably around 30% (+/-1%) total of one or more amphoteric (zwitterionic) surfactants. Preferred amphoteric surfactants include Velox BT-12C. Other amphoteric surfactants that may be used include betaines and betaine derivatives and fatty amine and fatty amine ethoxylate derivatives (such as alkyl betaine, sultaine, dihydroxyethyl glycinate, alkyl amidopropyl betaine, and aminopropionate) and amphoteric imadazoline derivatives (such as amphodiacetates, amphoacetates, amphocarboxylates, amphopropionate, amphodipropionate, and hydroxypropyl sulfonate). A mixture of amphoteric surfactants may also be used. Most preferably, only a single amphoteric surfactant is used.

A source of humic acid preferably comprises organic peat humus. The organic peat humus aids in binding H₂S, sulfur containing compounds, ammonia, and other related noxious compounds. It also contributes to the overall pH control in the wet scrubber. Organic peat humus typically has a pH of around 7.5-8.9 and is not a strongly acid compound like the mineral acids used in some prior art treatments. Preferably, a treatment composition comprises around 1-15%, more preferably around 6-12%, and more preferably around 10% (+/-1%) of organic peat humus. Although one or more mineral acids may also be used, most preferably, the compositions and methods do not include or use any mineral acids.

Optionally, one or more fragrances may be added to aid in masking odors and to identify the treatment composition as a cleaner/odor control product as it is added to the system. Preferably, a treatment composition comprises around 1-3%, more preferably around 1.5% (+/-0.1%) total of one or more fragrances. Preferred fragrances include lemon, peppermint, and orange scent.

An aqueous based solvent is also preferably used to combine the active ingredients and to provide optimized dissolution in the air scrubber water system. Preferably, a treatment composition comprises around 20-60%, more preferably around 25-35%, and most preferably around 29.5% (+/-0.5%) of an aqueous based solvent. Most preferably, DI water is used as the aqueous solvent.

The numerical values for ingredients or active concentration ranges in preferred embodiments herein described as a range specifically include any individual value within such ranges and any and all subset combinations within ranges, including subsets that overlap from one preferred range to a more preferred range and even if the specific subset of the range is not specifically described herein.

According to one particularly preferred embodiment, a treatment composition comprises the ingredients in Table 1 and active concentrations in the water of the wet scrubber when the composition is dosed at a preferred dosage of 125 ppm in the water:

**TABLE 1**

| **Ingredient** | **Composition Wt %** | **Active ppm in the water (when composition dosed at 125 ppm)** |
|---|---|---|
| Aqueous Solvent - DI Water | 29.5 | NA |
| Nonionic surfactant - ethoxylated alcohol surfactant | 4.0 | 5ppm |
| Amphoteric surfactant - coco alkyldimethyl betaine and cocoalkyldimethyl amine | 30.0 | 37.5ppm |
| Organic peat humus | 10.0 | 12.5ppm |
| Low Foam Nonionic Surfactant - Benzyl-capped ethoxylated C10-12-alcohols. | 25.0 | 31.25ppm |
| Fragrance | 1.5 | 1.9ppm |

Typical prior art treatments require the use of a defoamer/anti-foaming agent, such as FC101, to control foam created by the surfactants. Defoamers/antifoaming agents are typically separate products that have to be separately added to the air scrubber system; they are not included with the primary odor treatment composition. This adds additional steps and additional products that have to be shipped, stored, and handled. With preferred embodiments of the invention, it is not necessary to include in the treatment composition or separately add a defoamer/anti-foaming agent to the wet scrubber water system. Since the low foam nonionic surfactant aids in reducing foam, as shown in the lab test results discussed below, references herein to defoamer and/or anti-foaming agent do not include low foam nonionic surfactants. Additionally, a standard industry treatment is Component A/ReNew A that uses a surfactant and an enzyme. With preferred embodiments of the invention, it is not necessary to use any enzymes. These ingredients may be included or excluded from preferred treatment compositions. It is also preferred not to include any (1) strong acids (mineral acids), such as Hydrochloric acid, phosphoric acid, sulfuric acid, (2) biocides or oxidizers, such as chlorine products, and/or (3) Enzymes in the treatment compositions according to the invention.

The inclusion of the organic peat humus aids in maintaining the pH in the water in the wet scrubber at around 7 to 8.5. It is generally not necessary to add other pH control agents with the treatment composition, as the composition does not depend on strict control of the pH to be effective, it will be effective over a pH range of 7 to 8.5.

According to one preferred method of treating a wet scrubber to reduce odors and clean fill or packing material, a treatment composition comprising one or more surfactants and a source of humic-acid in a liquid formulation is dosed into the wet scrubber water. Most preferably, the treatment composition used is one according to a preferred embodiment of the invention. A preferred dosage rate provides a concentration for the composition in the water of the wet scrubber of around 50-1000 ppm, more preferably around 125 to 500 ppm. Preferred active concentrations in the water of the wet scrubber when the composition is added comprise:
(1) 5 to 20 ppm, more preferably 5 to 10 ppm, and most preferably around 5 ppm total of one or more regular nonionic surfactants;
(2) 37.5 to 150 ppm, more preferably 37.5 to 75 ppm, and most preferably around 37.5 ppm total of one or more amphoteric (zwitterionic) surfactants;
(3) 10 to 50 ppm, more preferably 10 to 30 ppm, and most preferably around 12.5 ppm of humic acid;
(4) optionally, but preferably, 31.25 to 125 ppm, more preferably 31.25 to 60 ppm, and most preferably around 31.25 ppm total of one or more low foam nonionic surfactants (in addition to ingredient 1); and
(5) optionally 1.9 to 7.6 ppm, more preferably 1.9 to 5 ppm, and most preferably around 1.9 ppm of a fragrance.

Again, the numerical values for ingredients or concentration ranges in preferred embodiments herein described as a range specifically include any individual value within such ranges and any and all subset combinations within ranges, including subsets that overlap from one preferred range to a more preferred range and even if the specific subset of the range is not specifically described herein.

Most preferably, the treatment composition is added to the make-up water for the wet scrubber through a chemical pump. The composition may also be added to any other convenient location within the wet scrubber's water system, such as a recirculating line or a sump.

According to other preferred embodiments, a concentration of the composition may be monitored by measuring a tracer in the composition or measuring COD in the water to calculate a concentration of the composition remaining in the water. Additionally, the composition may be periodically batch added or slowly continually added to the wet scrubber system to maintain a preferred concentration level.

According to still other preferred embodiments, a method of reducing odors in a wet air scrubber having a volume of water comprises the following steps: (1) adding a treatment composition to the water in the wet air scrubber to provide a concentration of the treatment composition of 50 to 1000 ppm in the volume of water, wherein the treatment composition comprises (a) one or more nonionic surfactants, (b) one or more amphoteric surfactant, and (c) a source of humic acid, wherein the amounts of these ingredients are according to preferred embodiments of the treatment compositions of the invention; (2) periodically adding additional treatment composition to maintain the concentration level within the 50 to 1000 ppm range or to maintain a concentration level for the actives in the treatment composition within the ranges described herein; (3) optionally, but preferably, not adding any separate defoamer to the water in the wet scrubber; and (4) optionally, but preferably, not adding any enzymes, biocides, oxidizers, chlorine compounds, or strong acids to the water in the air scrubber system as part of the treatment composition, but such may be added to the water in the air scrubber separately from the treatment composition if desired. According to another preferred embodiment, none of any one or any combination of the following are added to the water in the air scrubber while the treatment composition is present in the water in a concentration range of 50 ppm or greater: enzymes, biocides, oxidizers, chlorine compounds, strong acids, and/or defoamers.

According to another preferred embodiment of the composition and method, all ingredients in the treatment composition are pre-mixed in a single container for ease of shipping, handling, storage, and accurate dosing.

Compositions and methods according to preferred embodiments were tested in a lab scale air scrubber system to determine their efficacy compared to prior art treatments. The air scrubber was charged with 10 liters of tap water that was recirculated through a series of nozzles that sprayed the water over high surface area packing media. Gaseous contaminants (acetic acid) were introduced into the bottom of the packing material and the concentration in the headspace above the packing material was measured using Draeger tubes.

Four treatment compositions and two controls were used in the lab test. Two of the compositions tested are embodiments of the invention (Formula 1 and Formula 2) for comparison with two prior art, commercially available products currently used in air scrubber applications (Component A+B as one of those products and Component A+C as the other of those products). A water-only control ("Tap Water Only") and an air-only control ("No Water") were used in the lab scale tests.

Formula 1 according to a preferred embodiment of the invention comprises: DI water 54.5%, Tomadol 91-6 (a regular nonionic surfactant) 4%, Colateric CB (an amphoteric surfactant, same as used in Component A) 30%, Simply Lemon (fragrance) 1.5%, and CA 87500 (source of organic peat humus) 10%. The percentages are by weight of Formula 1. Formula 1 was dosed in the lab test at a concentration of 125 ppm. CA87500 is a product commercially available from Chem-Aqua that is used for sulfur removal in some scrubber applications. CA87500 comprises water (substrate), organic peat humus (active ingredient), potassium hydroxide at <1.0% (alkaline metal to extract desired compounds), and xanthan gum at <1.0% (suspension agent). The percentages are by weight of CA87500.

Formula 2 according to a preferred embodiment of the invention comprises: DI water 29.5%, Tomadol 91-6 (a regular nonionic surfactant) 4%, Colateric CB (an amphoteric surfactant, same as used in Component A) 30%, Simply Lemon (fragrance) 1.5%, CA 87500 (source of organic peat humus) 10%, and Triton DF-12 (a low foam nonionic surfactant, same as used in Component B) 25%. The percentages are by weight of Formula 2. Formula 2 was also dosed in the lab test at a concentration of 125 ppm.

The Component A+B and Component A+C products are commercially available ReNew^{®} products from Diversey that are industry standard treatment. These products are believed to be as described in U.S. Patent Application Publication 2012/0219480. The Component A is an amphoteric surfactant commercially known as Colateric CB with an enzyme; Component B is a low foam nonionic surfactant commercially known as Triton DF-12; and Component C is a pH control agent (citric acid). When Component A+B products were used in the test, the A component had to be separately added to the water from the B component. Similarly, when the Component A+C products were used in the test, the A component had to be separately added to the water from the C component. This is because these components are not suitable to be pre-mixed together into a single composition that can be stored as such. Component A was dosed for both combinations at a concentration of 125 ppm. Components B and C were each dosed for its respective combination with A at a concentration of 25 ppm. The total concentration for the two prior art combinations was 150 ppm, which was higher than the concentration of the Formulas 1 and 2 according to preferred embodiments of the invention.

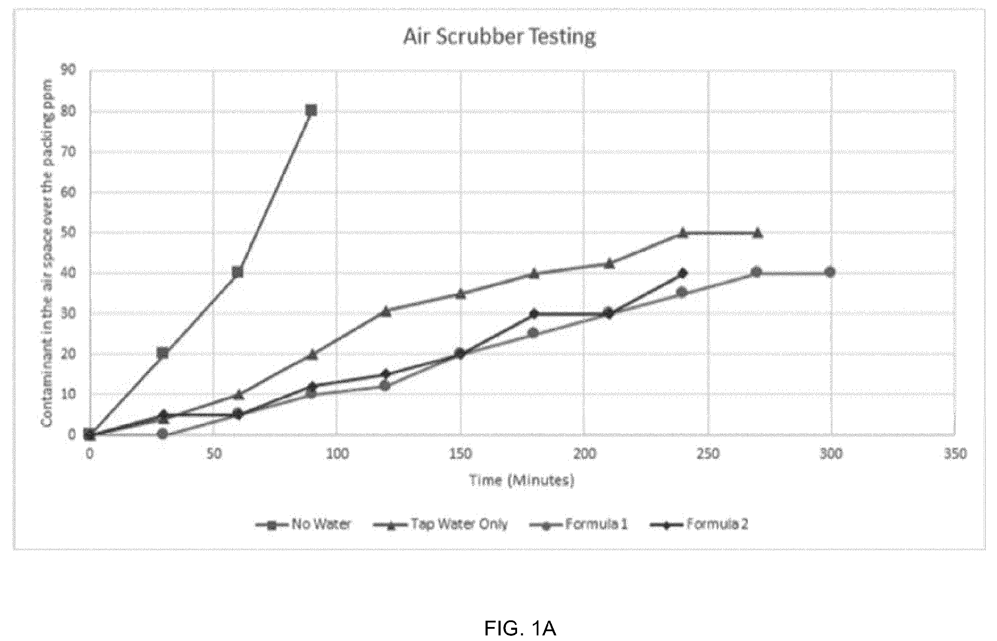

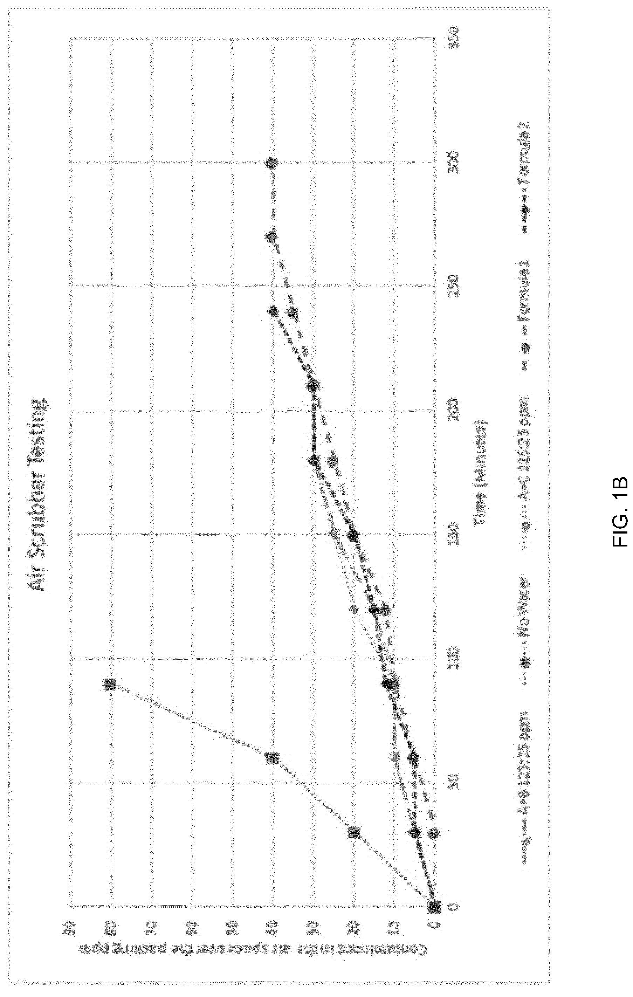

FIGS. 1A and 1B are graphs showing the contaminant (acetic acid) concentration (ppm) as a function of time for each of the four treatments and the two controls in the lab test. It can be seen from FIGS. 1A and 1B that:
1. In the air-only control (with no water added) there was a rapid increase in the contaminant in the air reaching 80ppm in less than 100 minutes. The air-only control is shown in squares on the graph lines in both FIGS. 1A and 1B.
2. The addition of water in the tap water-only control slowed the rate at which the contaminant was introduced into the air. It reached around 50 ppm at around 240 minutes (shown in FIG. 1A). This demonstrates that the lab scale scrubber is effective at transferring the contaminant from the air to the water. The tap water-only control is shown in triangles on the graph line in FIG. 1A.
3. Formulas 1 and 2 showed similar results with Formula 1 being slightly better than Formula 2 (best shown on FIG. 1A). Both Formulas 1 and 2 at 125 ppm further drops the amount of contaminant in the air space above the packing material compared to the water-only control. At around 240 minutes, the amount of contaminant in the air space was around 40 ppm for Formula 2 (which was the last reading for Formula 2) and around 36 ppm for Formula 1, both at least 10 ppm below the water-only control. By 300 minutes, Formula 1 still had not exceed 40 ppm of contaminant in the air space. However, as discussed below, Formula 2 showed significant improvement over Formula 1 with respect to foaming. Formula 1 is shown in circles on the graph line in FIG. 1A and also in larger circles and a larger dashed line on FIG. 1B. Formula 2 is shown in diamonds on the graph line in FIG. 1A and diamonds with a dashed line in FIG. 1B.
4. The two prior art products showed results that were similar to Formulas 1 and 2, although Formulas 1 and 2 were slightly better, even though the total concentration of the prior art product components (150 ppm total each) was greater than the concentration of Formulas 1 and 2 (125 ppm each). Additionally, Component A requires the presence of enzymes to achieve its odor reducing effectiveness, whereas preferred embodiments of the invention do not require any enzymes. No enzymes were used in Formulas 1 or 2. This is beneficial because enzymes are easily denatured by heat, pH, and chemical environment, all conditions that may be encountered in a wet air scrubber, rendering them inactive. As shown in FIG. 1B, the lines of the tested treatment products overlap. The Component A+B (shown in triangles), Component A+C (shown in circles with a shorter-dashed line), and Formula 2 (shown in diamonds) lines are overlapping until around 30 minutes, at which point Formula 2 stays level at about 5 pmm while the prior art treatments continue to climb to 10 ppm. The two prior art treatments remain overlapping at 10 ppm until around 90 minutes, at which point the Component A+C line climbs higher than the Component A+B line until they overlap again (and merge with Formula 2) at around 180 minutes and 30 ppm. The Component A+B, Component A+C, and Formula 2 lines remain overlapping at 30 ppm until around 210 minutes, which was the last reading for the prior art treatments. The Formula 1 treatment (shown in circles with a longer-dashed line) showed the best results.

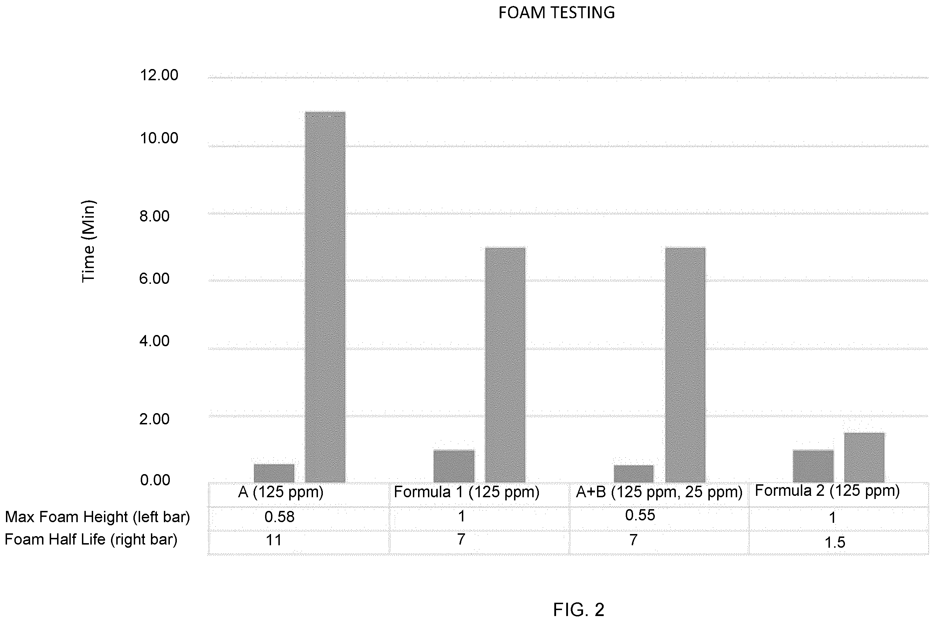

In addition to testing contaminant concentration in the air space above the lab scale air scrubber, foam levels and half-life were also tested. In the foam tests, Formulas 1 and 2 and prior art Component A+B products were the same as described above. However, the fourth treatment tested was prior art Component A only (without the pH control of C). The Component C was not included in this test because the pH control agent (citric acid) would not impact foam levels created by the surfactants and since it has to be separately added from Component A, the additional step was skipped for purposes of the test. FIG. 2 is a graph showing foam height and foam half-life for each of these four treatments in the lab test. The results in FIG. 2 shows the synergistic effect of using the low foaming surfactant with the other surfactant compounds according to preferred embodiments of the invention. The max foam height (which varied for each compound) was reached by all four tested products in under one minute. However, the foam half-life bars on the graph clearly show that when using the surfactant combination of Formula 2 according to a preferred embodiment of the invention, the foam breaks around 4 times faster than that observed with Formula 1 or the Component A+B product and around 6 times faster than Component A alone. This is important as it eliminates the need for additional defoamers/anti-foam agents to be added as an adjunct product, allowing the entire treatment to be performed from a single drum of treatment composition according to a preferred embodiment.

Two field trials, one at each of two different facilities, were also conducted to test a composition and method according to a preferred embodiment. Specifically, air scrubbers for high intensity applications like red meat and poultry odors were targeted in both trials to use as a worst-case scenario for performance. Both trials used a composition according to Formula 2 at a concentration of 100-200 ppm. The goal of the first field trial was to conduct a site assessment and observe the application and behavior of a composition and method according to a preferred embodiment in an active wet air scrubber servicing a rendering facility. The first field trial was successful, as the composition caused no adverse effects to the air scrubber system and was fed using existing equipment, so no changes to the air scrubber or its related treatment feeding equipment were needed to add the composition to the scrubber. The air scrubber in the first trial had previously been treated with ReNew^{®} for at least a month prior to the trial. Although quantitative analysis was not conducted, it was observed that the treatment in the first trial reduced odors from the air scrubber compared to a level of odors prior to the treatment.

The goal of the second field trial was to evaluate a composition and method according to a preferred embodiment in its ability to clean and/or keep clean the packing media in a wet air scrubber. The air scrubber in the second trial had previously been treated with with ReNew^{®} for at least a month prior to the trial. Although quantitative analysis was not conducted, it was observed that the treatment in the second trial resulted in a reduction in fouling on the surfaces of the scrubber and inline probes.

Compositions and methods according to preferred embodiments are effective at reducing contaminants in an air scrubber outlet/exhaust gas stream that create odors or are a nuisance. Compared to levels of contaminants in the outlet/exhaust gas stream without treatment (air only, no water), the compositions and methods can reduce the levels of contaminants by at least 40%, more preferably at least 50%. Compared to levels of contaminants in the outlet/exhaust gas stream with water only, the compositions and methods can reduce the levels of contaminants by at least 10%, more preferably at least 20% They are also effective at reducing foam levels in the air scrubber, as measured by foam half- life time, at least two times faster, preferably at least four times faster, more preferably at least six times faster compared to prior art treatments.

The compositions and methods of preferred embodiments of the invention may be used with any type of wet air scrubber, including a packed tower scrubber, a spray tower scrubber, an orifice scrubber, a venturi scrubber, a fiber-bed scrubber, an impingement-plate scrubber, a spray nozzle scrubber, a fluidized-bed scrubber, a packed-bed scrubber, multiple-stage scrubbers, baffle spray scrubber, a counter-flow scrubber, a crossflow scrubber, and combinations thereof. The compositions and methods of preferred embodiments of the invention may be used with any wet air scrubbers at a variety of factories or plants including, but not limited to, municipal wastewater plants, pet food plants, flavor and fragrance plants, rendering plants, food processing plants, breweries, and grain operations, such as corn processing. The compositions and methods of preferred embodiments of the invention may aid in controlling, reducing, and eliminating odors by aiding in the transfer of contaminants and odor causing substances from the air to the water in the scrubber, by cleaning packing or filling materials or media in the scrubber, and/or by eliminating the formation and/or growth of biofilm in wet air scrubbers.
All numerical values for amounts of ingredients, ratios, temperatures, pH values and other numeric values herein described as a range specifically include any individual value or ratio within such ranges and any and all subset combinations within ranges, including subsets that overlap from one preferred range to a more preferred range and even if the specific subset of the range is not specifically described herein.

## Claims

1. A treatment composition to reduce odors in a wet air scrubber, the treatment composition comprising:
one or more nonionic surfactants;
one or more amphoteric surfactants; and
a source of humic acid.

2. The treatment composition of claim 1 wherein the source of humic acid is organic peat humus.

3. The treatment composition of claim 2 further comprising an aqueous solvent.

4. The treatment composition of claim 3 comprising (1) 1-40% total of the one or more nonionic surfactants; (2) 20-40% total of the one or more amphoteric surfactants; and (3) 1-15% of the organic peat humus, the percentages by weight of the treatment composition.

5. The treatment composition of claim 3 wherein the one or more nonionic surfactants comprises 2-10% of one or more regular nonionic surfactants.

6. The treatment composition of claim 5 wherein the one or more nonionic surfactants further comprises 1-30% of one or more low foam nonionic surfactants.

7. The treatment composition of claim 6 further comprising a fragrance.

8. The treatment composition of claim 6 wherein the one or more regular nonionic surfactants comprises an ethoxylated alcohol or a nonionic ethylene oxide containing surfactant or both.

9. The treatment composition of claim 8 wherein the one or more low foam nonionic surfactants comprises a benzyl-capped *ethoxylated* C10-12-alcohol or a low cloud point nonionic surfactant or both.

10. The treatment composition of claim 1 wherein the treatment composition does not include any enzymes and does not include any mineral acids.

11. The treatment composition of claim 1 wherein the treatment composition does not include any oxidizers or chlorine compounds.

12. The treatment composition of claim 1 wherein the treatment composition is a pre-mixed liquid composition.

13. The treatment composition of claim 3 further comprising a fragrance.

14. A method of reducing odors in a wet air scrubber comprising a volume of water, the method comprising:
adding a treatment composition to the water in the wet air scrubber, wherein the treatment composition comprises (1) one or more nonionic surfactants, (2) one or more amphoteric surfactants, and (3) a source of humic acid.

15. The method of claim 14 wherein the source of humic acid is organic peat humus and wherein the treatment composition comprises (1) 1-40% total of the one or more nonionic surfactants; (2) 20-40% total of the one or more amphoteric surfactants; and (3) 1-15% of the organic peat humus, the percentages by weight of the treatment composition.

16. The method of claim 14 wherein the treatment composition is added in an amount that provides a concentration of the treatment composition of 50 to 1000 ppm in the volume of water.

17. The method of claim 15 wherein the treatment composition further comprises an aqueous solvent and all ingredients in the treatment composition are pre-mixed in a single container.

18. The method of claim 15 wherein the one or more nonionic surfactants comprises 2-10% of one or more regular nonionic surfactants and 1-30% of one or more low foam nonionic surfactants.

19. The method of claim 16 wherein no separate defoamer is added to the volume of water in the wet air scrubber.

20. The method of claim 19 wherein the treatment composition does not include any enzymes, oxidizers, or chlorine compounds.

21. The method of claim 15 wherein the treatment composition does not include any enzymes, oxidizers, or chlorine compounds.

22. The method of claim 14 wherein the one or more nonionic surfactants comprises one or more regular nonionic surfactants and of one or more low foam nonionic surfactants and wherein the source of humic acid is organic peat humus.

23. The method of claim 22 wherein the treatment composition is added in an amount that provides an active concentration of 5 to 20 ppm total of the one or more regular nonionic surfactants, 31.25 to 125 ppm ppm total of the one or more low foam nonionic surfactants, 37.5 to 150 ppm total of the one or more amphoteric surfactants, and 10-14 ppm of humic acid.

## Patentansprüche

1. Behandlungszusammensetzung zur Reduzierung von Gerüchen in einem Nassluftwäscher, wobei die Behandlungszusammensetzung umfasst:
ein oder mehrere nichtionische Tenside;
ein oder mehrere amphotere Tenside; und
eine Quelle für Huminsäure.

2. Behandlungszusammensetzung nach Anspruch 1, wobei die Quelle für Huminsäure organischer Torfhumus ist.

3. Behandlungszusammensetzung nach Anspruch 2 ferner umfassend ein wässriges Lösungsmittel.

4. Behandlungszusammensetzung nach Anspruch 3, umfassend (1) insgesamt 1-40 % des einen oder der mehreren nichtionischen Tenside; (2) insgesamt 20-40 % des einen oder der mehreren amphoteren Tenside; und (3) 1-15 % des organischen Torfhumus, wobei die Prozentangaben auf das Gewicht der Behandlungszusammensetzung bezogen sind.

5. Behandlungszusammensetzung nach Anspruch 3, wobei das eine oder die mehreren nichtionischen Tenside 2-10 % eines oder mehrerer regulärer nichtionischer Tenside umfassen.

6. Behandlungszusammensetzung nach Anspruch 5, wobei das eine oder die mehreren nichtionischen Tenside ferner 1-30 % eines oder mehrerer schaumarmer nichtionischer Tenside umfassen.

7. Behandlungszusammensetzung nach Anspruch 6 ferner umfassend einen Duftstoff.

8. Behandlungszusammensetzung nach Anspruch 6, wobei das eine oder die mehreren regulären nichtionischen Tenside einen ethoxylierten Alkohol oder ein nichtionisches ethylenoxidhaltiges Tensid oder beide umfassen.

9. Behandlungszusammensetzung nach Anspruch 8, wobei das eine oder die mehreren schaumarmen nichtionischen Tenside einen benzylverkappten ethoxylierten C10-12-Alkohol oder ein nichtionisches Tensid mit niedrigem Trübungspunkt oder beide umfassen.

10. Behandlungszusammensetzung nach Anspruch 1, wobei die Behandlungszusammensetzung keine Enzyme und keine Mineralsäuren enthält.

11. Behandlungszusammensetzung nach Anspruch 1, wobei die Behandlungszusammensetzung keine Oxidationsmittel oder Chlorverbindungen enthält.

12. Behandlungszusammensetzung nach Anspruch 1, wobei die Behandlungszusammensetzung eine vorgemischte flüssige Zusammensetzung ist.

13. Behandlungszusammensetzung nach Anspruch 3 ferner umfassend einen Duftstoff.

14. Verfahren zur Reduzierung von Gerüchen in einem Nassluftwäscher umfassend ein Wasservolumen, wobei das Verfahren umfasst:
Zugabe einer Behandlungszusammensetzung zu dem Wasser in dem Nassluftwäscher, wobei die Behandlungszusammensetzung (1) ein oder mehrere nichtionische Tenside, (2) ein oder mehrere amphotere Tenside und (3) eine Quelle für Huminsäure umfasst.

15. Verfahren nach Anspruch 14, wobei die Quelle für Huminsäure organischer Torfhumus ist und wobei die Behandlungszusammensetzung (1) insgesamt 1-40 % des einen oder der mehreren nichtionischen Tenside; (2) insgesamt 20-40 % des einen oder der mehreren amphoteren Tenside; und (3) 1-15 % des organischen Torfhumus umfasst, wobei die Prozentangaben auf das Gewicht der Behandlungszusammensetzung bezogen sind.

16. Verfahren nach Anspruch 14, wobei die Behandlungszusammensetzung in einer Menge zugesetzt wird, die eine Konzentration der Behandlungszusammensetzung von 50 bis 1000 ppm in dem Wasservolumen bereitstellt.

17. Verfahren nach Anspruch 15, wobei die Behandlungszusammensetzung ferner ein wässriges Lösungsmittel umfasst und alle Bestandteile der Behandlungszusammensetzung in einem einzigen Behälter vorgemischt werden.

18. Verfahren nach Anspruch 15, wobei das eine oder die mehreren nichtionischen Tenside 2 bis 10 % eines oder mehrerer regulärer nichtionischer Tenside und 1 bis 30 % eines oder mehrerer schaumarmer nichtionischer Tenside umfassen.

19. Verfahren nach Anspruch 16, wobei dem Wasservolumen im Nassluftwäscher kein separater Entschäumer zugesetzt wird.

20. Verfahren nach Anspruch 19, wobei die Behandlungszusammensetzung keine Enzyme, Oxidationsmittel oder Chlorverbindungen enthält.

21. Verfahren nach Anspruch 15, wobei die Behandlungszusammensetzung keine Enzyme, Oxidationsmittel oder Chlorverbindungen enthält.

22. Verfahren nach Anspruch 14, wobei das eine oder die mehreren nichtionischen Tenside ein oder mehrere reguläre nichtionische Tenside und ein oder mehrere schaumarme nichtionische Tenside umfassen und wobei die Quelle für Huminsäure organischer Torfhumus ist.

23. Verfahren nach Anspruch 22, wobei die Behandlungszusammensetzung in einer Menge zugesetzt wird, die eine aktive Konzentration von insgesamt 5 bis 20 ppm des einen oder der mehreren regulären nichtionischen Tenside, von insgesamt 31,25 bis 125 ppm des einen oder der mehreren schaumarmen nichtionischen Tenside, von insgesamt 37,5 bis 150 ppm des einen oder der mehreren amphoteren Tenside und von 10-14 ppm Huminsäure bereitstellt.

## Revendications

1. Composition de traitement pour réduire des odeurs dans un épurateur d'air humide, la composition de traitement comprenant :
un ou plusieurs tensioactifs non ioniques ;
un ou plusieurs tensioactifs amphotères ; et
une source d'acide humique.

2. Composition de traitement selon la revendication 1 dans laquelle la source d'acide humique est un humus de tourbe organique.

3. Composition de traitement selon la revendication 2 comprenant en outre un solvant aqueux.

4. Composition de traitement selon la revendication 3 comprenant (1) de 1 à 40 % au total du ou des tensioactifs non ioniques ; (2) de 20 à 40 % au total du ou des tensioactifs amphotères ; et (3) de 1 à 15 % de l'humus de tourbe organique, en pourcentages en poids de la composition de traitement.

5. Composition de traitement selon la revendication 3 dans laquelle le ou les tensioactifs non ioniques comprennent de 2 à 10 % d'un ou plusieurs tensioactifs non ioniques ordinaires.

6. Composition de traitement selon la revendication 5 dans laquelle le ou les tensioactifs non ioniques comprennent en outre de 1 à 30 % d'un ou plusieurs tensioactifs non ioniques faiblement moussants.

7. Composition de traitement selon la revendication 6 comprenant en outre un parfum.

8. Composition de traitement selon la revendication 6 dans laquelle le ou les tensioactifs non ioniques ordinaires comprennent un alcool éthoxylé ou un tensioactif contenant de l'oxyde d'éthylène non ionique ou les deux.

9. Composition de traitement selon la revendication 8 dans laquelle le ou les tensioactifs non ioniques faiblement moussants comprennent un alcool en C10 à C12 éthoxylé à coiffe benzyle ou un tensioactif à bas point de trouble ou les deux.

10. Composition de traitement selon la revendication 1 dans laquelle la composition de traitement ne comporte ni enzymes ni acides minéraux.

11. Composition de traitement selon la revendication 1 dans laquelle la composition de traitement ne comporte ni oxydants ni composés chlorés.

12. Composition de traitement selon la revendication 1 dans laquelle la composition de traitement est une composition liquide pré-mélangée.

13. Composition de traitement selon la revendication 3 comprenant en outre un parfum.

14. Procédé de réduction des odeurs dans un épurateur d'air humide comprenant un volume d'eau, le procédé comprenant :
l'ajout d'une composition de traitement dans l'eau de l'épurateur d'air humide, dans lequel la composition de traitement comprend (1) un ou plusieurs tensioactifs non ioniques, (2) un ou plusieurs tensioactifs amphotères et (3) une source d'acide humique.

15. Procédé selon la revendication 14 dans lequel la source d'acide humique est de l'humus de tourbe organique et dans lequel la composition de traitement comprend (1) de 1 à 40 % au total du ou des tensioactifs non ioniques ; (2) de 20 à 40 % au total du ou des tensioactifs amphotères ; et (3) de 1 à 15 % de l'humus de tourbe organique, en pourcentages en poids de la composition de traitement.

16. Procédé selon la revendication 14 dans lequel la composition de traitement est ajoutée en une quantité qui fournit une concentration de la composition de traitement de 50 à 1 000 ppm dans le volume d'eau.

17. Procédé selon la revendication 15 dans lequel la composition de traitement comprend en outre un solvant aqueux et tous les ingrédients de la composition de traitement sont pré-mélangés dans un récipient unique.

18. Procédé selon la revendication 15 dans lequel le ou les tensioactifs non ioniques comprennent de 2 à 10 % du ou des tensioactifs non ioniques ordinaires et de 1 à 30 % du ou des tensioactifs non ioniques faiblement moussants.

19. Procédé selon la revendication 16 dans lequel aucun suppresseur de mousse distinct n'est ajouté dans le volume d'eau dans l'épurateur d'air humide.

20. Procédé selon la revendication 19 dans lequel la composition de traitement ne comporte ni enzymes ni oxydants ni composés chlorés.

21. Procédé selon la revendication 15 dans lequel la composition de traitement ne comporte ni enzymes ni oxydants ni composés chlorés.

22. Procédé selon la revendication 14 dans lequel le ou les tensioactifs non ioniques comprennent un ou plusieurs tensioactifs non ioniques ordinaires et un ou plusieurs tensioactifs non ioniques faiblement moussants et dans lequel la source d'acide humique est de l'humus de tourbe organique.

23. Procédé selon la revendication 22 dans lequel la composition de traitement est ajoutée en une quantité qui fournit une concentration active de 5 à 20 ppm au total du ou des tensioactifs non ioniques ordinaires, de 31,25 à 125 ppm au total du ou des tensioactifs non ioniques faiblement moussants, de 37,5 à 150 ppm au total du ou des tensioactifs amphotères et de 10 à 14 ppm d'acide humique.
